# EUROPÄISCHE PATENTANMELDUNG

(11) **EP 2 329 854 A2**
(43) Veröffentlichungstag der Anmeldung: **08.06.2011**
(21) Anmeldenummer: 10190654.3
(22) Anmeldetag: 10.11.2010
(51) Int. Cl.: A61L 31/10, A61L 31/16

(54) **Implantatbeschichtung mit Nukleinsäuren**

(30) Priorität: 04.12.2009 US 266520 P
(71) Anmelder: Biotronik VI Patent AG, 6341 Baar (CH)
(72) Erfinder: Borck, Alexander, 91086, Aurachtal (DE); Gratz, Matthias, 91054, Erlangen (DE)
(74) Vertreter: Lindner-Vogt, Karin L.

(57) **Zusammenfassung**

Die vorliegende Erfindung bezieht sich auf ein Implantat mit einer Beschichtung oder einer Kavitätenfüllung enthaltend eine Nukleinsäure die i) die Expression mindestens eines Vertreters der Ras-Genfamilie mittels RNA interference inhibieren kann; oder ii) für eine Nukleinsäure kodiert, die die Expression mindestens eines Vertreters der Ras-Genfamilie mittels RNA interference inhibieren kann.

SEQUENCE LISTING

<110>  BIOTRONIK

<120>  Implantatbeschichtung mit Nukleinsäuren

<130>  09.056P-EP

<160>  12

<170>  PatentIn version 3.3

<210>  1
<211>  4461
<212>  DNA
<213>  Homo sapiens

<400>  1
gaaacgtccc gtgtgggagg ggcgggtctg ggtgcggcct gccgcatgac tcgtggttcg      60

gaggcccacg tggccggggc ggggactcag gcgcctgggg cgccgactga ttacgtagcg     120

ggcggggccg gaagtgccgc tccttggtgg gggctgttca tggcggttcc ggggtctcca     180

acatttttcc cggctgtggt cctaaatctg tccaaagcag aggcagtgga gcttgaggtt     240

cttgctggtg tgaaatgact gagtacaaac tggtggtggt tggagcaggt ggtgttggga     300

aaagcgcact gacaatccag ctaatccaga accactttgt agatgaatat gatcccacca     360

tagaggattc ttacagaaaa caagtggtta tagatggtga aacctgtttg ttggacatac     420

tggatacagc tggacaagaa gagtacagtg ccatgagaga ccaatacatg aggacaggcg     480

aaggcttcct ctgtgtattt gccatcaata atagcaagtc atttgcggat attaacctct     540

acagggagca gattaagcga gtaaaagact cggatgatgt acctatggtg ctagtgggaa     600

acaagtgtga tttgccaaca aggacagttg atacaaaaca agcccacgaa ctggccaaga     660

gttacgggat tccattcatt gaaacctcag ccaagaccag acagggtgtt gaagatgctt     720

tttacacact ggtaagagaa atacgccagt accgaatgaa aaaactcaac agcagtgatg     780

atgggactca gggttgtatg ggattgccat gtgtggtgat gtaacaagat acttttaaag 840

ttttgtcaga aaagagccac tttcaagctg cactgacacc ctggtcctga cttccctgga 900

ggagaagtat tcctgttgct gtcttcagtc tcacagagaa gctcctgcta cttccccagc 960

tctcagtagt ttagtacaat aatctctatt tgagaagttc tcagaataac tacctcctca 1020

cttggctgtc tgaccagaga atgcacctct tgttactccc tgttattttt ctgccctggg 1080

ttcttccaca gcacaaacac acctctgcca ccccaggttt ttcatctgaa aagcagttca 1140

tgtctgaaac agagaaccaa accgcaaacg tgaaattcta ttgaaaacag tgtcttgagc 1200

tctaaagtag caactgctgg tgattttttt tttcttttta ctgttgaact tagaactatg 1260

ctaatttttg gagaaatgtc ataaattact gttttgccaa gaatatagtt attattgctg 1320

tttggtttgt ttataatgtt atcggctcta ttctctaaac tggcatctgc tctagattca 1380

taaatacaaa aatgaatact gaattttgag tctatcctag tcttcacaac tttgacgtaa 1440

ttaaatccaa ctttcacagt gaagtgcctt tttcctagaa gtggtttgta gacttccttt 1500

ataatatttc agtggaatag atgtctcaaa aatccttatg catgaaatga atgtctgaga 1560

tacgtctgtg acttatctac cattgaagga aagctatatc tatttgagag cagatgccat 1620

tttgtacatg tatgaaattg gttttccaga ggcctgtttt ggggctttcc caggagaaag 1680

atgaaactga aagcacatga ataatttcac ttaataattt ttacctaatc tccacttttt 1740

tcataggtta ctacctatac aatgtatgta atttgtttcc cctagcttac tgataaacct 1800

aatattcaat gaacttccat ttgtattcaa atttgtgtca taccagaaag ctctacattt 1860

gcagatgttc aaatattgta aaactttggt gcattgttat ttaatagctg tgatcagtga 1920

ttttcaaacc tcaaatatag tatattaaca aattacattt tcactgtata tcatggtatc 1980

ttaatgatgt atataattgc cttcaatccc cttctcaccc caccctctac agcttccccc 2040

acagcaatag gggcttgatt atttcagttg agtaaagcat ggtgctaatg gaccagggtc 2100

acagtttcaa aacttgaaca atccagttag catcacagag aaagaaattc ttctgcattt 2160

gctcattgca ccagtaactc cagctagtaa ttttgctagg tagctgcagt tagccctgca 2220

aggaaagaag aggtcagtta gcacaaaccc tttaccatga ctggaaaact cagtatcacg 2280

tatttaaaca tttttttttc ttttagccat gtagaaactc taaattaagc caatattctc 2340

atttgagaat gaggatgtct cagctgagaa acgttttaaa ttctctttat tcataatgtt 2400

ctttgaaggg tttaaaacaa gatgttgata aatctaagct gatgagtttg ctcaaaacag 2460

gaagttgaaa ttgttgagac aggaatggaa aatataatta attgatacct atgaggattt 2520

ggaggcttgg cattttaatt tgcagataat accctggtaa ttctcatgaa aaatagactt 2580

ggataacttt tgataaaaga ctaattccaa aatggccact ttgttcctgt ctttaatatc 2640

taaatactta ctgaggtcct ccatcttcta tattatgaat tttcatttat taagcaaatg 2700

tcatattacc ttgaaattca gaagagaaga aacatatact gtgtccagag tataatgaac 2760

ctgcagagtt gtgcttctta ctgctaattc tgggagcttt cacagtactg tcatcatttg 2820

taaatggaaa ttctgctttt ctgtttctgc tccttctgga gcagtgctac tctgtaattt 2880

tcctgaggct tatcacctca gtcatttctt ttttaaatgt ctgtgactgg cagtgattct 2940

ttttcttaaa aatctattaa atttgatgtc aaattaggga gaaagatagt tactcatctt 3000

gggctcttgt gccaatagcc cttgtatgta tgtacttaga gttttccaag tatgttctaa 3060

gcacagaagt ttctaaatgg ggccaaaatt cagacttgag tatgttcttt gaatacctta 3120

agaagttaca attagccggg catggtggcc cgtgcctgta gtcccagcta cttgagaggc 3180

tgaggcagga gaatcacttc aacccaggag gtggaggtta cagtgagcag agatcgtgcc 3240

actgcactcc agcctgggtg acaagagaga cttgtctcca aaaaaaaagt tacacctagg 3300

tgtgaatttt ggcacaaagg agtgacaaac ttatagttaa aagctgaata acttcagtgt 3360

ggtataaaac gtggttttta ggctatgttt gtgattgctg aaaagaattc tagtttacct 3420

caaaatcctt ctctttcccc aaattaagtg cctggccagc tgtcataaat tacatattcc 3480

ttttggtttt tttaaaggtt acatgttcaa gagtgaaaat aagatgttct gtctgaaggc 3540

taccatgccg gatctgtaaa tgaacctgtt aaatgctgta tttgctccaa cggcttacta 3600

tagaatgtta cttaatacaa tatcatactt attacaattt ttactatagg agtgtaatag 3660

gtaaaattaa tctctatttt agtgggccca tgtttagtct ttcaccatcc tttaaactgc 3720

tgtgaatttt tttgtcatga cttgaaagca aggatagaga aacactttag agatatgtgg 3780

ggttttttta ccattccaga gcttgtgagc ataatcatat ttgctttata tttatagtca 3840

tgaactccta agttggcagc tacaaccaag aaccaaaaaa tggtgcgttc tgcttcttgt 3900

aattcatctc tgctaataaa ttataagaag caaggaaaat tagggaaaat attttatttg 3960

gatggtttct ataaacaagg gactataatt cttgtacatt atttttcatc tttgctgttt 4020

ctttgagcag tctaatgtgc cacacaatta tctaaggtat ttgttttcta taagaattgt 4080

tttaaaagta ttcttgttac cagagtagtt gtattatatt tcaaaacgta agatgatttt 4140

taaaagcctg agtactgacc taagatggaa ttgtatgaac tctgctctgg agggagggga 4200

ggatgtccgt ggaagttgta agacttttat ttttttgtgc catcaaatat aggtaaaaat 4260

aattgtgcaa ttctgctgtt taaacaggaa ctattggcct ccttggccct aaatggaagg 4320

gccgatattt taagttgatt attttattgt aaattaatcc aacctagttc tttttaattt 4380

ggttgaatgt tttttcttgt taaatgatgt ttaaaaaata aaaactggaa gttcttggct 4440

tagtcataat tcttatattc a 4461

<210> 2
<211> 5436
<212> DNA
<213> Homo sapiens

<400> 2
ggccgcggcg gcggaggcag cagcggcggc ggcagtggcg gcggcgaagg tggcggcggc 60

tcggccagta ctcccggccc ccgccatttc ggactgggag cgagcgcggc gcaggcactg 120

aaggcggcgg cggggccaga ggctcagcgg ctcccaggtg cgggagagag gcctgctgaa 180

aatgactgaa tataaacttg tggtagttgg agctggtggc gtaggcaaga gtgccttgac 240

gatacagcta attcagaatc attttgtgga cgaatatgat ccaacaatag aggattccta 300

caggaagcaa gtagtaattg atggagaaac ctgtctcttg gatattctcg acacagcagg 360

tcaagaggag tacagtgcaa tgagggacca gtacatgagg actggggagg gctttctttg 420

tgtatttgcc ataaataata ctaaatcatt tgaagatatt caccattata gagaacaaat 480

taaaagagtt aaggactctg aagatgtacc tatggtccta gtaggaaata aatgtgattt 540

gccttctaga acagtagaca caaaacaggc tcaggactta gcaagaagtt atggaattcc 600

ttttattgaa acatcagcaa agacaagaca gagagtggag gatgcttttt atacattggt 660

gagggagatc cgacaataca gattgaaaaa aatcagcaaa gaagaaaaga ctcctggctg 720

tgtgaaaatt aaaaaatgca ttataatgta atctgggtgt tgatgatgcc ttctatacat 780

tagttcgaga aattcgaaaa cataaagaaa agatgagcaa agatggtaaa aagaagaaaa 840

agaagtcaaa gacaaagtgt gtaattatgt aaatacaatt tgtacttttt tcttaaggca 900

tactagtaca agtggtaatt tttgtacatt acactaaatt attagcattt gttttagcat 960

tacctaattt ttttcctgct ccatgcagac tgttagcttt taccttaaat gcttatttta 1020

aaatgacagt ggaagttttt ttttcctcta agtgccagta ttcccagagt tttggttttt 1080

gaactagcaa tgcctgtgaa aaagaaactg aatacctaag atttctgtct tggggttttt 1140

ggtgcatgca gttgattact tcttattttt cttaccaatt gtgaatgttg gtgtgaaaca 1200

aattaatgaa gcttttgaat catccctatt ctgtgtttta tctagtcaca taaatggatt 1260

aattactaat ttcagttgag accttctaat tggtttttac tgaaacattg agggaacaca 1320

aatttatggg cttcctgatg atgattcttc taggcatcat gtcctatagt ttgtcatccc 1380

tgatgaatgt aaagttacac tgttcacaaa ggttttgtct cctttccact gctattagtc 1440

atggtcactc tccccaaaat attatatttt ttctataaaa agaaaaaaat ggaaaaaaat 1500

tacaaggcaa tggaaactat tataaggcca tttccttttc acattagata aattactata 1560

aagactccta atagcttttc ctgttaaggc agacccagta tgaaatgggg attattatag 1620

caaccatttt ggggctatat ttacatgcta ctaaattttt ataataattg aaaagatttt 1680

aacaagtata aaaaattctc ataggaatta aatgtagtct ccctgtgtca gactgctctt 1740

tcatagtata actttaaatc ttttcttcaa cttgagtctt tgaagatagt tttaattctg 1800

cttgtgacat taaaagatta tttgggccag ttatagctta ttaggtgttg aagagaccaa 1860

ggttgcaagg ccaggccctg tgtgaacctt tgagctttca tagagagttt cacagcatgg 1920

actgtgtccc cacggtcatc cagtgttgtc atgcattggt tagtcaaaat ggggagggac 1980

tagggcagtt tggatagctc aacaagatac aatctcactc tgtggtggtc ctgctgacaa 2040

atcaagagca ttgcttttgt ttcttaagaa aacaaactct tttttaaaaa ttacttttaa 2100

atattaactc aaaagttgag attttggggt ggtggtgtgc caagacatta attttttttt 2160

taaacaatga agtgaaaaag ttttacaatc tctaggtttg gctagttctc ttaacactgg 2220

ttaaattaac attgcataaa cacttttcaa gtctgatcca tatttaataa tgctttaaaa 2280

taaaaataaa aacaatcctt ttgataaatt taaaatgtta cttattttaa aataaatgaa 2340

gtgagatggc atggtgaggt gaaagtatca ctggactagg aagaaggtga cttaggttct 2400

agataggtgt cttttaggac tctgattttg aggacatcac ttactatcca tttcttcatg 2460

ttaaaagaag tcatctcaaa ctcttagttt ttttttttta caactatgta atttatattc 2520

catttacata aggatacact tatttgtcaa gctcagcaca atctgtaaat ttttaaccta 2580

tgttacacca tcttcagtgc cagtcttggg caaaattgtg caagaggtga agtttatatt 2640

tgaatatcca ttctcgtttt aggactcttc ttccatatta gtgtcatctt gcctccctac 2700

cttccacatg ccccatgact tgatgcagtt ttaatacttg taattcccct aaccataaga 2760

tttactgctg ctgtggatat ctccatgaag ttttcccact gagtcacatc agaaatgccc 2820

tacatcttat ttcctcaggg ctcaagagaa tctgacagat accataaagg gatttgacct 2880

aatcactaat tttcaggtgg tggctgatgc tttgaacatc tctttgctgc ccaatccatt 2940

agcgacagta ggatttttca aacctggtat gaatagacag aaccctatcc agtggaagga 3000

gaatttaata aagatagtgc tgaaagaatt ccttaggtaa tctataacta ggactactcc 3060

tggtaacagt aatacattcc attgttttag taaccagaaa tcttcatgca atgaaaaata 3120

ctttaattca tgaagcttac tttttttttt tggtgtcaga gtctcgctct tgtcacccag 3180

gctggaatgc agtggcgcca tctcagctca ctgcaacctc catctcccag gttcaagcga 3240

ttctcgtgcc tcggcctcct gagtagctgg gattacaggc gtgtgccact acactcaact 3300

aatttttgta tttttaggag agacggggtt tcaccctgtt ggccaggctg gtctcgaact 3360

cctgacctca agtgattcac ccaccttggc ctcataaacc tgttttgcag aactcattta 3420

ttcagcaaat atttattgag tgcctaccag atgccagtca ccgcacaagg cactgggtat 3480

atggtatccc caaacaagag acataatccc ggtccttagg tagtgctagt gtggtctgta 3540

atatcttact aaggcctttg gtatacgacc cagagataac acgatgcgta ttttagtttt 3600

gcaaagaagg ggtttggtct ctgtgccagc tctataattg ttttgctacg attccactga 3660

aactcttcga tcaagctact ttatgtaaat cacttcattg ttttaaagga ataaacttga 3720

ttatattgtt tttttatttg gcataactgt gattctttta ggacaattac tgtacacatt 3780

aaggtgtatg tcagatattc atattgaccc aaatgtgtaa tattccagtt ttctctgcat 3840

aagtaattaa aatatactta aaaattaata gttttatctg ggtacaaata aacaggtgcc 3900

tgaactagtt cacagacaag gaaacttcta tgtaaaaatc actatgattt ctgaattgct 3960

atgtgaaact acagatcttt ggaacactgt ttaggtaggg tgttaagact tacacagtac 4020

ctcgtttcta cacagagaaa gaaatggcca tacttcagga actgcagtgc ttatgagggg 4080

atatttaggc ctcttgaatt tttgatgtag atgggcattt ttttaaggta gtggttaatt 4140

acctttatgt gaactttgaa tggtttaaca aaagatttgt ttttgtagag attttaaagg 4200

gggagaattc tagaaataaa tgttacctaa ttattacagc cttaaagaca aaaatccttg 4260

ttgaagtttt tttaaaaaaa gctaaattac atagacttag gcattaacat gtttgtggaa 4320

gaatatagca gacgtatatt gtatcatttg agtgaatgtt cccaagtagg cattctaggc 4380

tctatttaac tgagtcacac tgcataggaa tttagaacct aacttttata ggttatcaaa 4440

actgttgtca ccattgcaca attttgtcct aatatataca tagaaacttt gtggggcatg 4500

ttaagttaca gtttgcacaa gttcatctca tttgtattcc attgattttt tttttcttct 4560

aaacattttt tcttcaaaca gtatataact ttttttaggg gatttttttt tagacagcaa 4620

aaactatctg aagatttcca tttgtcaaaa agtaatgatt tcttgataat tgtgtagtaa 4680

tgttttttag aacccagcag ttaccttaaa gctgaattta tatttagtaa cttctgtgtt 4740

aatactggat agcatgaatt ctgcattgag aaactgaata gctgtcataa aatgaaactt 4800

tctttctaaa gaaagatact cacatgagtt cttgaagaat agtcataact agattaagat 4860

ctgtgtttta gtttaatagt ttgaagtgcc tgtttgggat aatgataggt aatttagatg 4920

aatttagggg aaaaaaaagt tatctgcaga tatgttgagg gcccatctct ccccccacac 4980

ccccacagag ctaactgggt tacagtgttt tatccgaaag tttccaattc cactgtcttg 5040

tgttttcatg ttgaaaatac ttttgcattt ttcctttgag tgccaatttc ttactagtac 5100

tatttcttaa tgtaacatgt ttacctggaa tgtattttaa ctatttttgt atagtgtaaa 5160

ctgaaacatg cacattttgt acattgtgct ttcttttgtg ggacatatgc agtgtgatcc 5220

agttgttttc catcatttgg ttgcgctgac ctaggaatgt tggtcatatc aaacattaaa 5280

aatgaccact cttttaattg aaattaactt ttaaatgttt ataggagtat gtgctgtgaa 5340

gtgatctaaa atttgtaata tttttgtcat gaactgtact actcctaatt attgtaatgt 5400

aataaaaata gttacagtga caaaaaaaaa aaaaaa 5436

<210> 3

<211>  1061
<212>  DNA
<213>  Homo sapiens

<400>  3
tgccctgcgc ccgcaacccg agccgcaccc gccgcggacg gagcccatgc gcggggcgaa      60

ccgcgcgccc ccgcccccgc cccgccccgg cctcggcccc ggccctggcc ccgggggcag     120

tcgcgcctgt gaacggtggg gcaggagacc ctgtaggagg accccgggcc gcaggcccct     180

gaggagcgat gacggaatat aagctggtgg tggtgggcgc cggcggtgtg ggcaagagtg     240

cgctgaccat ccagctgatc cagaaccatt ttgtggacga atacgacccc actatagagg     300

attcctaccg gaagcaggtg gtcattgatg gggagacgtg cctgttggac atcctggata     360

ccgccggcca ggaggagtac agcgccatgc gggaccagta catgcgcacc ggggagggct     420

tcctgtgtgt gtttgccatc aacaacacca agtcttttga ggacatccac cagtacaggg     480

agcagatcaa acgggtgaag gactcggatg acgtgcccat ggtgctggtg gggaacaagt     540

gtgacctggc tgcacgcact gtggaatctc ggcaggctca ggacctcgcc cgaagctacg     600

gcatccccta catcgagacc tcggccaaga cccggcaggg agtggaggat gccttctaca     660

cgttggtgcg tgagatccgg cagcacaagc tgcggaagct gaaccctcct gatgagagtg     720

gccccggctg catgagctgc aagtgtgtgc tctcctgacg cagcacaagc tcaggacatg     780

gaggtgccgg atgcaggaag gaggtgcaga cggaaggagg aggaaggaag gacggaagca     840

aggaaggaag gaagggctgc tggagcccag tcaccccggg accgtgggcc gaggtgactg     900

cagaccctcc cagggaggct gtgcacagac tgtcttgaac atcccaaatg ccaccggaac     960

cccagccctt agctcccctc ccaggcctct gtgggccctt gtcgggcaca gatgggatca    1020

cagtaaatta ttggatggtc ttgaaaaaaa aaaaaaaaaa a                        1061


<210>  4
<211>  19
<212>  RNA

<213>  artificial

<220>
<223>  siRNA N-Ras sense

<400>  4
gucauuugcg gauauuaac                                                    19

<210>  5
<211>  19
<212>  RNA
<213>  artificial

<220>
<223>  siRNA N-Ras antisense

<400>  5
guuaauaucc gcaaaugac                                                    19

<210>  6
<211>  47
<212>  DNA
<213>  artificial

<220>
<223>  DNA Sequence encoding shRNA N-Ras

<400>  6
gtcatttgcg gatattaact tcaagagagt taatatccgc aaatgac                     47

<210>  7
<211>  19
<212>  RNA
<213>  artificial

<220>
<223>  siRNA K-Ras sense

<400>  7
ggcauacuag uacaagugg                                                    19

<210>  8
<211>  19
<212>  RNA
<213>  artificial

<220>
<223>  siRNA K-Ras antisense

<400>  8
ccacuuguac uaguaugcc                                                    19



<210>  9
<211>  47
<212>  DNA
<213>  artificial

<220>
<223>  DNA Sequence encoding shRNA K-Ras

<400>  9
ggcatactag tacaagtggt tcaagagacc acttgtacta gtatgcc                     47



<210>  10
<211>  19
<212>  RNA
<213>  artificial

<220>
<223>  siRNA H-Ras sense

<400>  10
uauaagcugg ugguggugg                                                    19



<210>  11
<211>  19
<212>  RNA
<213>  artificial

<220>
<223>  siRNA H-Ras antisense

<400>  11

ccaccaccac cagcuuaua                                                  19

<210>  12
<211>  47
<212>  DNA
<213>  artificial

<220>
<223>  DNA Sequence encoding shRNA H-Ras

<400>  12
ggagcgatga cggaatatat ttgtgtagta tattccgtca tcgctcc                    47

## Beschreibung

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z.B. Stents), zur Befestigung und temporären Fixierung von Gewebeimplantaten und Gewebstransplantationen, aber auch zu orthopädischen Zwecken, zum Beispiel als Nagel, Platte oder Schraube. Häufig ist nur eine temporäre Stütz- beziehungsweise Haltefunktion bis zum Abschluss des Heilungsprozesses oder Stabilisierung des Gewebes notwendig beziehungsweise erwünscht. Um Komplikationen zu vermeiden, die aus dem dauerhaften Verbleib der Implantate im Körper resultieren, müssen die Implantate entweder wieder operativ entfernt werden oder sie bestehen aus einem Werkstoff, der allmählich im Körper abgebaut wird, das heißt biokorrodierbar ist. Die Zahl biokorrodierbarer Werkstoffe auf der Basis von Polymeren oder Legierungen ist stetig gewachsen. So sind unter anderem biokorrodierbare Metalllegierungen der Elemente Magnesium, Eisen und Wolfram bekannt. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch Bildgebende Verfahren, wie z.B. durch Röntgenuntersuchungen erfolgen.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe für Stents umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder Ti-A16Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität und damit eine Verbesserung der Restenoserate erreichen lässt, wenn Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Die Wirkstoffe können zum Beispiel auf der Implantatoberfläche in Form einer Beschichtung bereitgestellt werden. Es ist bekannt, dass eine Unterdrückung der Ras-vermittelten Signaltransduktion ein vielversprechender Weg zur Hemmung der Zellproliferation ist. Problematisch ist, dass es im Ras-System ein hohes Maß an Redundanz gibt. Die Zelle kann mehrere verschiedene Proteine der Ras-Familie exprimieren, die mindestens teilweise die Hemmung eines bestimmten Vertreters der Ras-Familie kompensieren können. Aufgrund dieser Redundanz ist eine wirksame Beeinflussung der Neointimaproliferation sehr schwierig.

Aufgabe der vorliegenden Erfindung ist es mindestens einen der Nachteile des Standes der Technik zu vermindern oder zu vermeiden.

Diese Aufgabe wird gelöst durch Bereitstellung eines Implantats mit einer Beschichtung oder einer Kavitätenfüllung enthaltend mindestens eine Nukleinsäure die
i) die Expression mindestens eines Vertreters der Ras-Genfamilie mittels RNA interference inhibieren kann; oder
ii) für eine Nukleinsäure kodiert, die die Expression mindestens eines Mitglieds der Ras-Genfamilie mittels RNA interference inhibieren kann.

Die erfindungsgemäße Lösung sieht vor, dass gezielt die Expression und Funktion mindestens eines Vertreters der Ras-Familie, bevorzugt aber mehrerer Vertreter der Ras-Familie parallel, mittels der RNA interference (RNAi) Technologie, in den das Implantat umgebenden Zellen gehemmt wird und somit eine unerwünschte Neointimabildung vermindert oder ganz vermieden werden kann. Ist das Implantat ein Stent, so lässt sich durch die erfindungsgemäße Beschichtung oder Kavitätenfüllung einer Restenose vorbeugen oder die Ausbildung einer Restenose verlangsamen. Dazu weist das Implantat eine Beschichtung und/oder eine Kavitätenfüllung auf, welche Nukleinsäuren enthält, die entweder selbst in der Lage sind über einen RNAi-Effekt die Expression mindestens eines Ras-Vertreters in einer Zielzelle zu inhibieren, oder für eine solche Nukleinsäure kodieren, sodass nach Aufnahme durch eine Zielzelle die entsprechende Nukleinsäure transkribiert werden kann und das Transkript über einen RNAi-Effekt seine inhibitorische Wirkung entfalten kann. Ein Vorteil des vorgestellten RNAi-Systems ist, dass sich so Inhibitoren verschiedener Ras-Vertreter wirksam in einer Beschichtung kombinieren lassen, da sich die Inhibitoren in ihren physikochemischen Eigenschaften sehr ähneln und sich aufgrund der hohen Spezifität der Nukleinsäuren mit RNAi-Wirkung gezielt gewünschte Ras-Vertreter hemmen lassen ohne nennenswerte Nebenwirkungen auf die Expression oder Funktion anderer Gene und/oder Proteine erwarten zu müssen. Nach erfolgter Applikation des erfindungsgemäßen Implantats, kann die Nukleinsäure aus der Beschichtung oder der Kavitätenfüllung austreten, von umliegenden Zellen aufgenommen werden und in diesen Zielzellen die gewünschte RNAi-Wirkung entfalten.

Bevorzugt besteht das Implantat ganz oder in Teilen aus einem biokorrodierbaren metallischen Werkstoff. Dieser biokorrodierbare Werkstoff ist bevorzugt eine Magnesiumlegierung.

Bevorzugt ist das erfindungsgemäße Implantat ein Stent.

Vorzugsweise weist das Implantat einen metallischen Grundkörper auf. Insbesondere besteht der metallische Grundkörper aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/1, CaCl₂ 0,2 g/1, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen ― abgestimmt auf das zu erwartende Korrosionsverhalten ― von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Erfindungsgemäß weist das Implantat eine Beschichtung oder eine Kavitätenfüllung auf, die eine Nukleinsäure enthält. Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Eine Schichtdicke liegt vorzugsweise im Bereich von 1 nm bis 100 µm, besonders bevorzugt 300 nm bis 15 µm. Die Beschichtung kann direkt auf die Implantatoberfläche aufgetragen werden. Die Verarbeitung kann nach Standardverfahren für die Beschichtung erfolgen. Es können einschichtige, aber auch mehrschichtige Systeme (zum Beispiel so genannte base coat -, drug coat - oder top coat - Schichten) erstellt werden. Die Beschichtung kann unmittelbar auf dem Grundkörper des Implantats aufgebracht sein oder es sind weitere Schichten dazwischen vorgesehen.

Alternativ kann die Nukleinsäure Bestandteil einer Kavitätenfüllung sein. Die Kavität befindet sich in der Regel an der Oberfläche des Implantats. Bei Stents mit einem biodegradierbaren Grundkörper kann die Kavität auch im Innern des Grundkörpers angeordnet sein, so dass die Freisetzung der Nukleinsäure erst nach Freilegung erfolgt. Die Kavität kann wahlweise offen oder durch eine weitere Beschichtung abgedeckt sein.

Verfahren zur Beschichtung von Implantaten und zur Anbringung von Kavitätenfüllungen auf Implantaten sind dem Fachmann bekannt.

Neben der Verwendung erfindungsgemäßer Nukleinsäuren kann die Beschichtung oder Kavitätenfüllung weitere Bestandteile enthalten, insbesondere eine polymere Trägermatrix, in die die Nukleinsäure in fein dispergierter Form eingebettet ist. Mit anderen Worten, das Implantat weist eine Beschichtung oder Kavitätenfüllung auf, die eine polymere, bevorzugt organische Trägermatrix mit eingebetteten Nukleinsäuren umfasst oder daraus besteht. Die Trägermatrix kann insbesondere weitere pharmazeutische Wirkstoffe, Röntgenmarker oder Magnetresonanzmarker enthalten.

Insbesondere kann die Beschichtung oder Kavitätenfüllung des erfindungsgemäßen Implantats die Nukleinsäure in Form von mit Nukleinsäure beladenen Nanopartikeln enthalten. Als Nanopartikel werden hierbei Partikel bezeichnet deren mittlerer Durchmesser < 1 µm ist. Bevorzugt werden Nanopartikel eingesetzt, die mit siRNA-Molekülen beladen sind. Für die Herstellung von mit Nukleinsäuren beladenen Nanopartikeln eignen sich Polykatione, wie: Poly-L-Lysin (PLL), Chitosan, Poly(diallyldimethyl-amonium chlorid) (Poly-DADMAC), Dimethylaminoethyl acrylat, Dimethylaminoethyl methacrylat, Dimethylamoniumpropylmethacrylamid, Dimethylaminomethylacrylamid, Acryloxyethyltrimethylammonium hlorid, Methacyloxyethyltrimethylammonium chlorid, Methacrylamidopropyltrimethylammoniumchlorid, 3-Methacryloxy(2-hydroxy)propyltrimethylammonium chlorid, (3-Acrylamido-3-methyl)butyltrimethylammonium chlorid, Polyetylenamine, Polyetylenimine, kationische dendritische Polymere, Diethylaminoethyl-Dextran, Polyamidoamin, Carboxymethylcellulose, Polyester der Milchsäure und deren Co-Polymerisate mit Glycolsäure wie PGLA. Zur Verminderung zytotoxischer Eigenschaften der Polykatione können diese durch Einsatz von Blockcopolymeren mit Polyethylenglycol verträglicher gestaltet werden. Hierzu kann das Polykation, z.B. PLL als PLL-hydrobromid, mit beispielsweise einem N-hydroxysuccinimidylester eines methoxyterminierten Polyethylenglycols umgesetzt werden.

Vorzugsweise können die Nukleinsäure beladenen Nanopartikel folgendermaßen hergestellt werden. Die Nukleinsäure, bevorzugt in Form von RNA, besonders bevorzugt in Form von doppelsträngiger RNA, ganz besonders bevorzugt in Form von siRNA oder shRNA, wird in eine Polykationenlösung gegeben, bevorzugt unter rühren. Dabei richtet sich die Konzentration an Polykationen nach der eingesetzten Menge an Nukleinsäure. Ein geeignetes Verhältnis kann der Fachmann ohne weiteres durch Routineversuche bestimmen. Geeignete Verhältnisse von positiven Ladungen (z. B. Aminogruppen) des Polykations zu den Phosphatgruppen der Nukleinsäure (N+/P-) liegen im Bereich von 2:1 bis 10:1, bevorzugt ist ein Verhältnis von 3:1. Bei einem Überschuss von positiven Ladungen, insbesondere bei einem Verhältnis von 3:1, werden Nanopartikel von < 200 nm erhalten, die besonders günstige Transfektionseigenschaften besitzen.

Das erfindungsgemäße Implantat weist eine Beschichtung oder eine Kavitätenfüllung enthaltend mindestens eine Nukleinsäure auf. Unter "Nukleinsäure" werden dabei einzelne Nukleotide und Polymere aus Nukleotiden verstanden. Der Begriff "Nukleinsäure" umfasst sowohl DNA als auch RNA und DNA/RNA-Hybride. Weist eine Nukleinsäure eine bestimmte Sequenz auf, so wird diese Sequenz in 5' ― 3'-Richtung angegeben. Erfindungsgemäß kann eine Nukleinsäure sowohl natürlich vorkommende Nukleotide wie A, G, T, C, U aufweisen, eine Nukleinsäure kann aber auch ein oder mehrere modifizierte und oder künstliche Nukleotide oder Nukleotidanaloga aufweisen. Im beiliegenden Sequenzprotokoll sind die Nukleinsäuresequenzen entweder für den Fall angegeben, dass es sich bei der Nukleinsäuresequenz um ein RNA-Molekül handelt oder dass es sich um ein DNA-Molekül handelt. Selbstverständlich umfassen die angegebenen Sequenzen auch die entsprechende Sequenz in der jeweils anderen Nukleinsäureklasse. Die Sequenzen mit den SEQ ID Nrs 4 bis 12 umfassen erfindungsgemäß sowohl RNA, DNA als auch RNA/DNA-Hybride mit der angegebenen Sequenz. Dabei wird das Nukleotid U (Uracil) einer RNA-Sequenz durch das Nukleotid T (Thymin) ersetzt, um eine DNA-Sequenz darzustellen und umgekehrt.

Die mindestens eine Nukleinsäure der erfindungsgemäßen Beschichtung oder Kavitätenfüllung kann entweder selbst die Expression mindestens eines Vertreters der Ras-Genfamilie mittels RNA interference (RNAi) inhibieren oder für eine Nukleinsäure kodieren, die die Expression mindestens eines Vertreters der Ras-Genfamilie mittels RNAi inhibieren kann.

Die Ras-Genfamilie umfasst eine Familie von Genen die für Proteine mit GTPase-Funktion kodiert, die bei einer Reihe von zellulären Signaltransduktionen eine entscheidende Rolle spielen, insbesondere bei der Vermittlung der Zellproliferation. Vertreter der Ras-Genfamilie zeichnen sich dadurch aus, das sie für Proteine kodieren, welche eine sog. G-Domäne und eine sog. C-terminale Membran-targeting-Region (CAAX-COOH, auch bekannt als CAAX box), welche lipid-modifiziert sein kann, aufweisen. Eine aktuelle Zusammenfassung der Ras-Genfamilie ist beispielsweise zu finden in Wennerberg K, Rossman KL, Der CJ (2005) The Ras superfamily at a glance, J. Cell. Sci. 118: 843-6. Insbesondere umfasst die Ras-Genfamilie folgende Vertreter: DIRAS1; DIRAS2; DIRAS3; ERAS; GEM; H-RAS; K-RAS; MRAS; NKIRAS1; NKIRAS2; N-RAS; RALA; RALB; RAP1A; RAP1B; RAP2A; RAP2B; RAP2C; RASD1; RASD2; RASL10A; RASL10B; RASL11A, RASL11B; RASL12; REM1; REM2; RERG; RERGL; RRAD; RRAS; RRAS2. Bevorzugte Vertreter der Ras-Genfamilie sind N-Ras mit der SEQ ID Nr. 1, K-Ras mit der SEQ ID Nr. 2 und/oder H-Ras mit der SEQ ID Nr. 3, wobei hier jeweils die cDNA-Sequenzen der jeweiligen Vertreter der Ras-Genfamilie angegeben sind. Bevorzugt weist das erfindungsgemäße Implantat eine Beschichtung oder eine Kavitätenfüllung auf, die eine Nukleinsäure enthält, die die Expression mindestens eines der Gene N-Ras mit der SEQ ID Nr. 1, K-Ras mit der SEQ ID Nr. 2 und/oder H-Ras mit der SEQ ID Nr. 3 inhibieren kann oder eines Gens, dessen cDNA zu ≥ 80%, bevorzugt ≥ 90%, besonders bevorzugt ≥5% identisch ist mit einer der Sequenzen mit der SEQ ID Nr. 1, 2 und/oder 3.

Erfindungsgemäß kann die mindestens eine Nukleinsäure der Beschichtung oder der Kavitätenfüllung selbst die Expression mindestens eines Vertreters der Ras-Genfamilie mittels RNAi inhibieren. Es ist bekannt, dass sich die Genexpression posttranskriptional durch die Anwesenheit doppelsträngiger RNA-Fragmente, die Bereiche aufweisen, die homolog sind zur Sequenz der mRNA des zu unterdrückenden Gens, inhibieren lässt. Dieser Vorgang wird als RNA interference oder RNAi bezeichnet. Einen Überblick über die derzeitige Verwendung der RNAi-Technologie findet sich in Wadhwa et al. (2004), Know-how of RNA interference and its application in research and therapy, Mutat. Res. 567(1):71-84.

Nukleinsäuren, die in der RNAi-Technologie eingesetzt werden können, umfassen bevorzugt doppelsträngige RNA-Moleküle, die eine Sequenz aufweisen, die zu einer Sequenz des zu inhibierenden Gens homolog sind, d.h. hinsichtlich sense- und antisense-Strang mit dem betreffenden Sequenzabschnitts des zu inhibierenden Gens übereinstimmen. Für die Zwecke der vorliegenden Erfindung umfasst der Begriff "doppelsträngig" sowohl eine intramolekulare als auch eine intermolekulare Doppelstrangbildung. Bevorzugt liegt der homologe Bereich im Bereich des Transksripts des zu inhibierenden Gens, also im Bereich der mRNA (bzw. cDNA) des zu inhibierenden Gens. Der homologe Bereich liegt bevorzugt im kodierenden Bereich oder im 5'- oder 3'-UTR-Bereich (UTR=untranslated region) der mRNA des zu inhibierenden Gens. Die erfindungsgemäßen Nukleinsäuren sind bevorzugt doppelsträngige oder teilweise gepaarte/hybridiserte RNA-Moleküle.

Erfindungsgemäß kann die Nukleinsäure als Doppelstrang glatte (blunt ends) oder überstehende (sticky ends) Enden aufweisen. Als besonders wirksam haben sich doppelsträngige Nukleinsäuren erwiesen, die am 3'-Ende von jedem Strang einen Überhang von 1 bis 6, vorzugsweise 1 oder 2 Nukleotiden aufweisen. Bei den überstehenden Nukleotiden handelt es sich vorzugsweise um 2'-Desoxynukleotide, besonders bevorzugt 2'-Desoxythymidinreste. Durch die Verwendung der 2'-Desoxynukleotide lassen sich die Kosten der RNA-Synthese reduzieren und die Widerstandsfähigkeit der RNA gegenüber dem Nukleaseabbau erhöhen. Die überstehenden Nukleotide müssen nicht zwangsläufig zur Zielsequenz homologe Nukleotide sein. Bevorzugt sind Nukleinsäuren mit kurzen Überständen, insbesondere von 2 Nukleotiden, bei denen die überstehenden Nukleotide des antisense-Strangs der doppelsträngigen Nukleinsäure zur Zielsequenz komplementär sind. Als besonders wirksam haben sich Nukleinsäuren erwiesen, die zu einem solchen Abschnitt des zu inhibierenden Gens und insbesondere der entsprechenden doppelsträngigen mRNA/cDNA homolog sind, dessen sense-Strang 5'-seitig durch zwei Adenosinreste (A) und 3'-seitig durch zwei Thymidinreste (T), einen Guanosin- (G) und einen Cytosinrest (C) oder einen Thymidin- und einen Cytidinrest (C) begrenzt wird. Der durch AA und TT, AA und GC bzw. AA und TC begrenzte Abschnitt weist vorzugsweise eine Länge von 19 bis 21, insbesondere 19 Nukleotiden auf und hat demnach die allgemeine Form AA(N₁₉₋₂₁)TT, AA(N₁₉₋₂₁)GC oder AA(N₁₉₋₂₁)TC, wobei N für ein beliebiges Nukleotid steht. Weiter bevorzugt sind Nukleinsäuren, die zu einem Abschnitt des zu inhibierenden Gens bzw. der entsprechenden doppelsträngigen cDNA oder mRNA komplementär sind, der die allgemeine Form AA(N₁₉) bis AA(N₂₁) hat. Hierbei sind Nukleinsäuren, die zu dem N₁₉₋₂₁-Fragment der genannten Bereiche homolog sind, besonders bevorzugt. Die besonders bevorzugten doppelsträngigen Nukleinsäuren weisen somit eine Länge von 19 bis 21 Basenpaaren auf, wobei die diese doppelsträngigen Nukleinsäuren bildenden Einzelstränge 3'-seitig vorzugsweise jeweils zwei zusätzliche 2'-Desoxynukleotide, insbesondere zwei 2'-Desoxythymidinreste aufweisen, so dass die dsRNA 19 bis 21 Basenpaare und pro Strang zwei überstehende 2'-Desoxynukleotide umfasst. Sollte das zu inhibierende Gen keinen Bereich der Form AA(N₁₉₋₂₁) enthalten, wird nach Bereichen der Form NA(N₁₉₋₂₁) oder einem beliebigen Fragment der Form N₁₉₋₂₁ gesucht. N₁₉₋₂₁-Fragmente, die z.B. von AA und TT begrenzt werden, sind zwar bevorzugt, grundsätzlich sind erfindungsgemäß jedoch alle doppelsträngigen Nukleinsäuren geeignet, die zu der Zielsequenz mindestens zu > 80%, bevorzugt zu ≥ 90%, besonders bevorzugt zu ≥ 95%, ganz besonders bevorzugt zu 100% identisch sind.

Nukleinsäuren, die selbst die Expression eines Vertreters der Ras-Genfamilie mittels RNAi inhibieren können, sind bevorzugt doppelsträngig, weisen zumindest einen Anteil an RNA auf oder bestehen aus RNA und können eine Länge aufweisen, die der mRNA bzw. cDNA des jeweiligen zu inhibierenden Gens entspricht oder kürzer sein. Bevorzugt weisen diese Nukleinsäuren eine Länge von 15 bis 49 Nukleotiden, besonders bevorzugt von 17 bis 30 Nukleotiden und ganz besonders bevorzugt von 10 bis 23 Nukleotiden auf. Erfindungsgemäß kann es sich bei der mindestens einen Nukleinsäure um ein siRNA-Molekül (small interfereing RNA) handeln. Dabei handelt es sich um ein doppelsträngiges RNA-Molekül mit einer Länge von 19 bis 25 Nukleotiden, welches eine Sequenz aufweist oder daraus besteht, die homolog ist zu einem Bereich der mRNA des Gens welches inhibiert werden soll (wie oben bereits beschriebenen). Alternativ kann die mindestens eine Nukleinsäure auch ein shRNA-Molekül sein (small hairpin RNA). Ein shRNA-Molekül weist zwei Sequenzbereiche auf, die zueinander revers komplementär sind und miteinander intramolekular einen Doppelstrang ausbilden können. Diese revers komplementären Bereiche sind durch einen Bereich voneinander getrennt, der keinen intramolekularen Doppelstrang bilden kann und bei Ausbildung eines Doppelstrangs der ersten beiden Bereiche als sog. Loop verbleibt. Die revers komplementären Bereiche weisen eine Sequenz auf, die homolog ist zu einem Bereich der mRNA des Gens welches inhibiert werden soll (wie oben bereits beschriebenen).

Erfindungsgemäß kann die Beschichtung oder die Kavitätenfüllung des Implantats eine Nukleinsäure enthalten, die für eine Nukleinsäure kodiert, die die Expression mindestens eines Vertreters der Ras-Genfamilie mittels RNAi inhibieren kann. Diese Nukleinsäure ist bevorzugt eine DNA-Nukleinsäure und kann neben der Sequenz, die für eine Nukleinsäure kodiert, die die Expression mindestens eines Vertreters der Ras-Genfamilie mittels RNAi inhibieren kann, weitere Bestandteile und Sequenzen umfassen. Bevorzugt umfasst diese Nukleinsäure regulatorische Elemente, die eine Expression der kodierten Nukleinsäure erlaubt. Dazu kann die Nukleinsäure einen Promoter aufweisen, z.B. einen U6 Promoter. Die Nukleinsäure kann in Form eines Vektors vorliegen. Dabei kann es sich um einen Plasmid-Vektor handeln oder um einen viralen Vektor, wie z.B. einen Vektor adenoviraler oder lentiviraler Abstammung. Der Vektor kann weitere funktionale Elemente oder Sequenzen umfassen, die sowohl der Expression der kodierten Nukleinsäure dienen, z.B. Promoter, Enhancer, etc, als auch solche, die nicht direkt der Expression der kodierten Nukleinsäure dienen, z.B. Selektionsmarker, Replikationsstellen, etc..

Bevorzugt weist das erfindungsgemäße Implantat eine Beschichtung oder Kavitätenfüllung auf, enthaltend mindestens eine Nukleinsäure, die eine Sequenz oder deren reverses Komplement umfasst oder daraus besteht, wobei die Sequenz ausgewählt ist aus Sequenzen mit der SEQ ID Nr. 4, 5, 6, 7, 8, 9, 10, 11 und/oder 12 oder aus Sequenzen die ≥ 80%, bevorzugt ≥ 90%, besonders bevorzugt ≥ 95%, identisch sind mit SEQ ID Nr. 4, 5, 6, 7, 8, 9, 10, 11 und/oder 12.

Bevorzugt weist die Beschichtung oder die Kavitätenfüllung mehrere verschiedene Nukleinsäuren auf, wobei mindestens zwei Nukleinsäuren die Expression unterschiedlicher Vertreter der Ras-Genfamilie inhibieren können oder für solche Nukleinsäuren kodieren. Besonders bevorzugt können die mindestens zwei verschiedenen Nukleinsäuren die Expression von jeweils unterschiedlichen Genen ausgewählt aus N-Ras mit der SEQ ID Nr. 1, K-Ras mit der SEQ ID Nr. 2 und/oder H-Ras mit der SEQ ID Nr. 3 inhibieren. Dazu kann die Beschichtung oder Kavitätenfüllung des erfindungsgemäßen Implantats mindestens zwei verschiedene Nukleinsäuren aufweisen, die jeweils eine Sequenz enthalten oder daraus bestehen ausgewählt aus:
i) N-Ras inhibierenden Sequenzen mit der SEQ ID Nr. 4, 5, 6 oder N-Ras inhibierende Sequenzen die ≥ 80%, bevorzugt ≥ 90%, besonders bevorzugt ≥ 95%, identisch sind mit SEQ ID Nr. 4, 5 oder 6;
ii) K-Ras inhibierenden Sequenzen mit der SEQ ID Nr. 7, 8, 9 oder K-Ras inhibierende Sequenzen die ≥ 80%, bevorzugt ≥ 90%, besonders bevorzugt ≥ 95%, identisch sind mit SEQ ID Nr. 7, 8 oder 9; und/oder
iii) H-Ras inhibierenden Sequenzen mit der SEQ ID Nr. 10, 11, 12 oder H-Ras inhibierende Sequenzen die ≥ 80%, bevorzugt ≥ 90%, besonders bevorzugt ≥ 95%, identisch sind mit SEQ ID Nr. 10, 11 oder 12;
wobei die Sequenz der ersten der zwei verschiedenen Nukleinsäuren nicht aus derselben Gruppe i), ii) oder iii) ausgewählt ist, wie die Sequenz der zweiten.

Bevorzugt weist die Beschichtung oder Kavitätenfüllung des erfindungsgemäßen Implantats eine Nukleinsäure auf, die aus einem siRNA-Molekül umfassend mindestens eine der Sequenzen mit der SEQ ID Nr 4, 5, 7, 8, 10 oder 11 und dem entsprechenden reversen Komplement dazu, oder einem shRNA-Molekül mit einer Sequenz der SEQ ID Nrs 6, 9 oder 12 gebildet ist.

Die Sequenzen mit der SEQ ID Nr. 4 und 5 können zusammen ein siRNA-Molekül bilden, welches geeignet ist die Expression des Gens N-Ras mit der SEQ ID Nr. 1 zu inhibieren und können als solches siRNA-Molekül die Nukleinsäure in der Beschichtung oder Kavitätenfüllung des erfindungsgemäßen Implantats bilden. Die Sequenz mit der SEQ ID Nr. 6 kann ein shRNA-Molekül zur Inhibition des Gens N-Ras mit der SEQ ID Nr. 1 darstellen und kann als solches die Nukleinsäure in der Beschichtung oder Kavitätenfüllung des erfindungsgemäßen Implantats bilden.

Die Sequenzen mit der SEQ ID Nr. 7 und 8 können zusammen ein siRNA-Molekül bilden, welches geeignet ist die Expression des Gens K-Ras mit der SEQ ID Nr. 2 zu inhibieren und können als solches siRNA-Molekül die Nukleinsäure in der Beschichtung oder Kavitätenfüllung des erfindungsgemäßen Implantats bilden. Die Sequenz mit der SEQ ID Nr. 9 kann ein shRNA-Molekül zur Inhibition des Gens K-Ras mit der SEQ ID Nr. 2 darstellen und kann als solches die Nukleinsäure in der Beschichtung oder Kavitätenfüllung des erfindungsgemäßen Implantats bilden.

Die Sequenzen mit der SEQ ID Nr. 10 und 11 können zusammen ein siRNA-Molekül bilden, welches geeignet ist die Expression des Gens H-Ras mit der SEQ ID Nr. 3 zu inhibieren und können als solches siRNA-Molekül die Nukleinsäure in der Beschichtung oder Kavitätenfüllung des erfindungsgemäßen Implantats bilden. Die Sequenz mit der SEQ ID Nr. 12 kann ein shRNA-Molekül zur Inhibition des Gens H-Ras mit der SEQ ID Nr. 3 darstellen und kann als solches die Nukleinsäure in der Beschichtung oder Kavitätenfüllung des erfindungsgemäßen Implantats bilden.

Verfahren zur Herstellung oben genannter Nukleinsäuren sind dem Fachmann bekannt.

Die vorliegende Erfindung bezieht sich auch auf Nukleinsäuren enthaltend oder bestehend aus einer Sequenz mit der SEQ ID Nr. 4, 5, 6, 7, 8, 9, 10, 11 und/oder 12 oder aus einer Sequenz, die ≥ 80%, bevorzugt ≥90%, besonders bevorzugt ≥ 95%, identisch ist mit SEQ ID Nr. 4, 5, 6, 7, 8, 9, 10, 11 und/oder 12, oder deren reverses Komplement zur Restenose-Prävention und/oder -Inhibition.

Nachfolgend wird die Erfindung anhand von Ausführungsbeispielen näher erläutert.

Die si- bzw. shRNA sollen in einer Art und Weise auf den Implantaten aufgebracht sein, die eine lokale Freisetzung und damit eine lokale Aufnahme in die Zellen gewährleistet.
1. Beschichtung mit si-/shRNA in Matrix, aus der die si-/shRNA eluiert. Wobei hier die ganze Bandbreite natürlicher und synthetischer, sowie der degradablen als auch der nichtabbaubaren Polymere einsetzbar ist.
2. Beschichtung mit si-/shRNA in Kavitäten. Wobei die si-/shRNA wahlweise in eine Matrix eingebettet ist und die Kavitäten wahlweise offen oder abgedeckt (Topcoat o.ä.) sind. Hier sind vor allem Hydrogele anwendbar, die die RNA in Mengen zischen 2 -50 % (m/m) vorzugsweise 10-25 % enthalten. Durch Quellung und / oder Diffusion können die Substanzen die Kavitäten verlassen, wobei die Substanz auch als Nanopartikel (siehe unten) aus dem Gel freigesetzt werden können.
3. Beschichtung mit siRNA-Mischung in Nanopartikeln.
4. Freisetzung von siRNA Mischungen in Nanopartikeln über geeignete Kathetersysteme.

Im Folgenden werden Ausführungsbeispiele für den Einbau der RNA Sequenzen in Nanopartikel gebracht.

Mischung von RNA mit Polyethylenimin: Das positiv geladene Polymer gibt mit den negativ geladenen RNA Molekülen in Wasser unlösliche Niederschläge, die bei entsprechender Rührgeschwindigkeit (300 UPM) und Zusatz von Detergentien wie 0,1 % Triton X100 als Nanopartikel ausfallen.

Alternativ kann statt des Polyethylenimin Polylysin eingesetzt werden, welches ebenfalls ein Polykation darstellt.

Chitosan ist ein weiteres Beispiel für ein Polykation. 5 mg Chitosan werden dazu in 1 ml einer 2% Essigsäurelösung gegeben. Zu dieser Lösung werden 1ml RNA Lösung (5,5 mg/ml) gegeben und dabei stark gevortext. Der feine Niederschlag wird in PBS Puffer 50 mM aufgenommen und weiterverarbeitet.

Neben diesen hydrophilen Polymeren sind auch hydrophobe Polymere wie PLLA, PLDLA, PLGA einsetzbar. Hier sollte über ein besonderes Verfahren z.B. eine Wasser /Öl/Wasser (W/Ö/W) Emulsion oder eine (W/Ö/Ö) Emulsion gearbeitet werden, um die rein wasserlösliche RNA in ein wasserunlösliches Polymer zu überführen (einzufangen).

### Beispiel einer W/Ö/W Emulsion:

### Erste Emulsion

PLLA L210 0,1-0,2 g in 100 ml Chloroform lösen. Zugabe von 1-2 ml RNA Lösung 5mg/ml. Die Zweiphasenmischung wird stark gerührt, Ultra-turrax 10.000 UPM. Das Polymer befindet sich in der organischen Phase und die RNA in der wässrigen.

### Zweite Emulsion

Zur ersten Emulsion wird nur 100 ml Wasser gegeben, welches ein Tensid Triton X100 0,2 % enthält. Die RNA liegt verkapselt im Polymer vor, in der Wasserphase ist nahezu nichts gelöst.

### Aufarbeitung:

Nach dem Abziehen des Lösungsmittels können die Polymer/RNA Nanopartikel erhalten werden.

Die so hergestellten Nanopartikel werden direkt in eine Hydrogelmatrix suspendiert oder in Kavitäten verfüllt. Die Nanopartikel, insbesondere die mit positivem Ladungsüberschuss an der Oberfläche, sind geeignet, die Nukleinsäureinformation in den Zellkern zu schleusen.

## Patentansprüche

1. Implantat mit einer Beschichtung oder einer Kavitätenfüllung enthaltend eine Nukleinsäure die
i) die Expression mindestens eines Vertreters der Ras-Genfamilie mittels RNA interference inhibieren kann; oder
ii) für eine Nukleinsäure kodiert, die die Expression mindestens eines Vertreters der Ras-Genfamilie mittels RNA interference inhibieren kann.

2. Implantat nach Anspruch 1, **dadurch gekennzeichnet, dass** das Implantat ganz oder in Teilen aus einem biokorrodierbaren metallischen Werkstoff besteht.

3. Implantat nach einem der vorhergehenden Ansprüche, **dadurch gekennzeichnet, dass** der biokorrodierbare metallische Werkstoff eine Magnesiumlegierung ist.

4. Implantat nach einem der vorhergehenden Ansprüche, **dadurch gekennzeichnet, dass** die Nukleinsäure die Expression mindestens eines der Gene N-Ras mit der SEQ ID Nr. 1, K-Ras mit der SEQ ID Nr. 2, H-Ras mit der SEQ ID Nr. 3 und/oder eines Gens, dessen cDNA zu ≥80%, bevorzugt ≥90%, besonders bevorzugt ≥95% identisch ist mit einer der Sequenzen mit der SEQ ID Nr. 1, 2 und/oder 3, inhibieren kann.

5. Implantat nach einem der vorhergehenden Ansprüche, **dadurch gekennzeichnet, dass** die Nukleinsäure eine Sequenz oder deren reverses Komplement umfasst oder daraus besteht, wobei die Sequenz ausgewählt ist aus Sequenzen mit der SEQ ID Nr. 4, 5, 6, 7, 8, 9, 10, 11 und/oder 12 oder aus Sequenzen die ≥80%, bevorzugt ≥90%, besonders bevorzugt ≥95%, identisch sind mit SEQ ID Nr. 4, 5, 6, 7, 8, 9, 10, 11 und/oder 12.

6. Implantat nach einem der vorhergehenden Ansprüche, **dadurch gekennzeichnet, dass** die Beschichtung oder die Kavitätenfüllung des Implantats mindestens zwei verschiedene Nukleinsäuren aufweist, wobei die beiden verschiedenen Nukleinsäuren die Expression unterschiedlicher Vertreter der Ras-Genfamilie inhibieren können.

7. Implantat nach einem der vorhergehenden Ansprüche, **dadurch gekennzeichnet, dass** die Beschichtung oder die Kavitätenfüllung des Implantats mindestens zwei verschiedene Nukleinsäuren aufweist, die jeweils eine Sequenz enthalten oder daraus bestehen ausgewählt aus:
i) N-Ras inhibierenden Sequenzen mit der SEQ ID Nr. 4, 5, 6 oder N-Ras inhibierende Sequenzen die ≥80%, bevorzugt ≥90%, besonders bevorzugt ≥95%, identisch sind mit SEQ ID Nr. 4, 5 oder 6;
ii) K-Ras inhibierenden Sequenzen mit der SEQ ID Nr. 7, 8, 9 oder K-Ras inhibierende Sequenzen die ≥80%, bevorzugt ≥90%, besonders bevorzugt ≥95%, identisch sind mit SEQ ID Nr. 7, 8 oder 9; und/oder
iii) H-Ras inhibierenden Sequenzen mit der SEQ ID Nr. 10, 11, 12 oder H-Ras inhibierende Sequenzen die ≥80%, bevorzugt ≥90%, besonders bevorzugt ≥95%, identisch sind mit SEQ ID Nr. 10, 11 oder 12;
wobei die jeweilige Sequenz der zwei verschiedenen Nukleinsäuren nicht aus derselben Gruppe i), ii) oder iii) ausgewählt ist.

8. Implantat nach einem der vorhergehenden Ansprüche, **dadurch gekennzeichnet, dass** die Nukleinsäure ein siRNA-Molekül bestehend aus einem Doppelstrang gebildet aus einer der Sequenzen SEQ ID Nrs 4, 5, 7, 8, 10 oder 11 und dem entsprechenden reversen Komplement dazu oder ein shRNA-Molekül mit einer Sequenz der SEQ ID Nrs 6, 9 oder 12 ist.

9. Implantat nach einem der Ansprüche 1 bis 7, **dadurch gekennzeichnet, dass** die Nukleinsäure eine DNA ist.

10. Implantat nach einem der vorhergehenden Ansprüche, **dadurch gekennzeichnet, dass** die Nukleinsäure einen Vektor umfasst.

11. Implantat nach einem der vorhergehenden Ansprüche, **dadurch gekennzeichnet, dass** die Beschichtung oder die Kavitätenfüllung des Implantats die Nukleinsäure in Form von mit Nukleinsäure beladenen Nanopartikeln enthält.

12. Implantat nach einem der vorhergehenden Ansprüche, **dadurch gekennzeichnet, dass** die Nukleinsäure in eine organische Matrix eingebettet ist, die als Beschichtung oder Kavitätenfüllung auf dem Implantat aufgebracht ist.

13. Implantat nach einem der vorhergehenden Ansprüche, **dadurch gekennzeichnet, dass** das Implantat ein Stent ist.

14. Nukleinsäure enthaltend oder bestehend aus einer Sequenz mit der SEQ ID Nr. 4, 5, 6, 7, 8, 9, 10, 11 und/oder 12 oder aus einer Sequenz, die ≥80%, bevorzugt ≥90%, besonders bevorzugt ≥95%, identisch ist mit SEQ ID Nr. 4, 5, 6, 7, 8, 9, 10, 11 und/oder 12, oder deren reverses Komplement zur Restenose-Prävention und/oder - Inhibition.
